(19) Europäisches Patentamt
European Patent Office
Office européen des brevets

(11) Veröffentlichungsnummer: **0 024 492**
**B1**

(12) **EUROPÄISCHE PATENTSCHRIFT**

(45) Veröffentlichungstag der Patentschrift: **05.05.82**

(21) Anmeldenummer: **80103483.6**

(22) Anmeldetag: **21.06.80**

(51) Int. Cl.³: **C 07 C 51/60**, C 07 C 63/15, C 07 C 63/22, C 07 C 63/24, C 07 C 63/30, C 07 C 63/38 // B01J27/00

(54) **Verfahren zur Herstellung aromatischer Dicarbonsäuredichloride und ihre Verwendung bei der Herstellung von Polykondensaten.**

(30) Priorität: **03.07.79 DE 2926779**

(43) Veröffentlichungstag der Anmeldung: **11.03.81 Patentblatt 81/10**

(45) Bekanntmachung des Hinweises auf die Patenterteilung: **05.05.82 Patentblatt 82/18**

(84) Benannte Vertragsstaaten: **BE DE FR GB IT NL**

(56) Entgegenhaltungen:
**JOURNAL OF ORGANIC CHEMISTRY, Band 43, Nr. 4, 17. Februar 1978, A.C.S. Seiten 591-595 CH. RAV-ACHA et al.: "Dipolar Micelles. 6. Catalytic effects of betaine-like micelles on the hydrolysis of substituted phenyl esters»**

**CHEMICAL ABSTRACTS, Band 75, Nr. 4, 26. Juli 1971, Seite 47, Nr. 21 707z Columbus, Ohio, USA**

(73) Patentinhaber: **BAYER AG, Zentralbereich Patente, Marken und Lizenzen, D-5090 Leverkusen 1, Bayerwerk (DE)**

(72) Erfinder: **Freitag, Dieter, Dr., Hasenheide 10, D-4150 Krefeld (DE)**
Erfinder: **Schmidt, Manfred, Dr., 1020 North-Third-Street Mobay Chemical Corp., New Martinsville 26155 (US)**

**EP 0 024 492 B1**

Anmerkung: Innerhalb von neun Monaten nach der Bekanntmachung des Hinweises auf die Erteilung des europäischen Patents im Europäischen Patentblatt kann jedermann beim Europäischen Patentamt gegen das erteilte europäische Patent Einspruch einlegen. Der Einspruch ist schriftlich einzureichen und zu begründen. Er gilt erst als eingelegt, wenn die Einspruchsgebühr entrichtet worden ist (Art. 99(1) Europäisches Patentübereinkommen).

ACTORUM AG.

# Verfahren zur Herstellung aromatischer Dicarbonsäuredichloriden und ihre Verwendung bei der Herstellung von Polykondensaten.

Die Erfindung betrifft ein Einstufen-Verfahren zur Herstellung von hochreinen polykondensationsfähigen aromatischen Dicarbonsäuredichloriden.

Die Erzeugung von aliphatischen und aromatischen Säurechloriden durch Reaktion einer Carbonsäure mit Phosgen ist in den US-PS 3 184 506, 3 544 626, 3 544 627, 3 547 960 sowie in den DE-OS 2 400 007 und 2 321 122 beschrieben. Nach diesen Verfahren werden als Reaktionsprodukte dunkelgefärbte Carbonsäurechloride in einer Reinheit von 96 bis 99% erhalten. Aromatische Dicarbonsäuredichloride dieses geringen Reinheitsgrades können nicht direkt nach dem Zweiphasengrenzflächenpolykondensationsverfahren zur Herstellung von hochmolekularen Polykondensaten, wie aromatischen Polyamiden oder aromatischen Polyestern, eingesetzt werden. Ihr Gehalt an nicht oder nur halbseitig umgesetzten Dicarbonsäuren stört die Polykondensation, verursacht unerwünschten Kettenabbruch und ergibt Polymerisate mit endständigen Carboxylgruppen. Die so hergestellten aromatischen Dicarbonsäuredichloride sind durch Verunreinigungen dunkel gefärbt und enthalten durch Umsetzung mit den Katalysatoren entstandene störende Carbamidsäurechloride (vergl. Chem. Ref. 1973, Vol. 73, Nr. 1, Seite 77 oder Angewandte Chemie (1974), Jahrg. 1962, Nr. 21, Seite 864).

Um farblose Dicarbonsäuredichloride zu erhalten, müssen die Rohprodukte durch Umkristallisation oder Destillation gereinigt werden. Dies erfordert zusätzlichen Aufwand und vermindert die Ausbeute; bei aromatischen Dicarbonsäuredichloriden besteht die Gefahr einer spontanen Zersetzung.

Gegenstand der Erfindung ist ein Einstufen-Verfahren zur Herstellung von reinen aromatischen Dicarbonsäuredichloriden durch Umsetzung einer aromatischen Dicarbonsäure mit Phosgen in Anwesenheit eines Katalysators und gegebenenfalls in einem Lösungsmittel und/oder Verdünnungsmittel, das dadurch gekennzeichnet ist, dass man als Katalysatoren Betaine verwendet. Die so erhaltenen aromatischen Dicarbonsäuredichloride sind praktisch farblos und enthalten neben den eingesetzten Katalysatoren 0,1% Verunreinigungen oder weniger, so dass sie ohne Nachreinigung zur Herstellung von farblosen, hochmolekularen Polykondensaten eingesetzt werden können.

Die nach dem erfindungsgemässen Verfahren als Katalysatoren eingesetzten Betaine stören beispielsweise die Herstellung von aromatischen Polyestern nicht, da sie einerseits als Phasentransferkatalysatoren die Umsetzung der aromatischen Dicarbonsäuredichloride mit Bisphenolen nach dem Zweiphasengrenzflächenpolykondensationsverfahren beschleunigen und andererseits in alkalisch wässrigem Medium löslich sind, so dass sie leicht durch die Waschprozesse des Herstellungsverfahrens von der organischen Lösung des Polykondensates abgetrennt werden können.

Als erfindungsgemässe Katalysatoren sind Betaine der Struktur (I), (II) oder (III) geeignet:

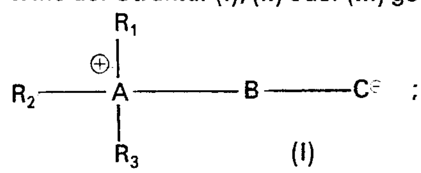

(I)

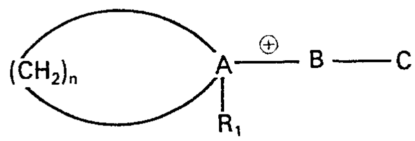

(II)

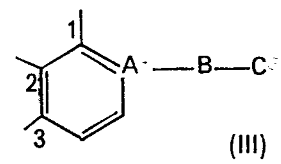

(III)

wobei

A = N oder P,

C = $-SO_3^{\ominus}$, $-COO^{\ominus}$

B = einen $C_1$-$C_2$-Alkylenrest, einen $C_6$-$C_{16}$-Cycloalkylen-, Aryl- oder Alkylarylrest,

$R_1$, $R_2$, $R_3$ gleich oder verschieden, einen $C_1$-$C_{12}$-Alkylrest, einen $C_6$-$C_{15}$-Aryl- oder Alkylaryl- oder Aralkylrest bedeuten können,

und in Struktur (II)n = 4 oder 5,

und in Struktur (III) der das Heteroatom A enthaltende aromatische Ring gegebenenfalls durch 1 bis 4 $C_1$-$C_4$-Alkylreste oder durch 1 bis 4 Halogenatome wie F, Cl oder Br substituiert sein kann oder durch einen weiteren aromatischen Ring in 1-2 Stellung oder 2-3 Stellung anelliert sein kann.

Folgende Einzelverbindungen sind beispielsweise geeignet:

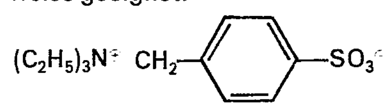

(1);

$(C_2H_5)_3N^{\oplus}-(CH_2)_4-SO_3^{\ominus}$ (2);

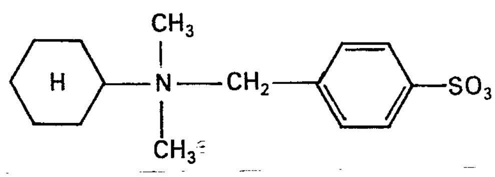

(3);

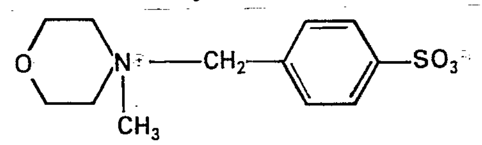

(4);

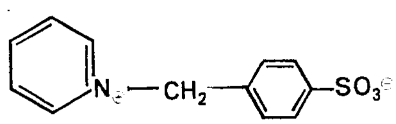

(5);

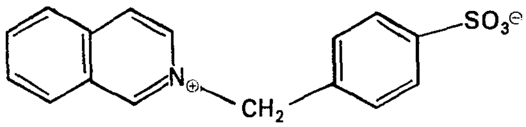
(6);

$(CH_3)_3N^{\oplus}$—$CH_2$—$CO_2^{\ominus}$ (7);

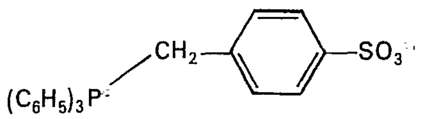
(8);

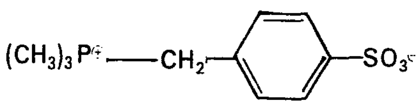
(9);

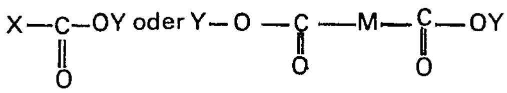
mit

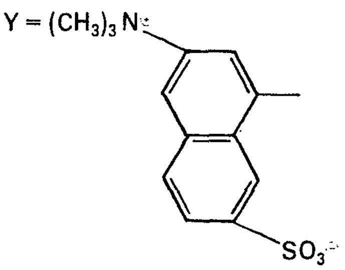

X = $C_1$–$C_{10}$-Alkyl oder $C_6$–$C_{10}$-Aryl,
M = eine Einfachbindung, ein $C_2$–$C_8$-Alkylenrest oder ein $C_6$–$C_{10}$-Arylenrest.

(1) p-(Triethylammonium-methyl)-benzol-sulfobetain,
(2) Triethyl-butylammonium-4-sulfobetain,
(3) p-(Trimethyl-cyclohexylammonium)-benzol-sulfobetain,
(4) p-(Dimethyl-morpholino)-benzol-sulfobetain,
(5) p-(Pyridinium-methyl)-benzol-sulfobetain,
(6) p-(Isochinolinium-methyl)-benzol-sulfobetain,
(7) Trimethylglycin = «Betain»,
(8) p-(Triphenylphosphonium-methyl)-benzol-sulfobetain,
(9) p-(Tetramethylphosphonium)-benzol-sulfobetain.

Besonders geeignet sind folgende Einzelverbindungen:

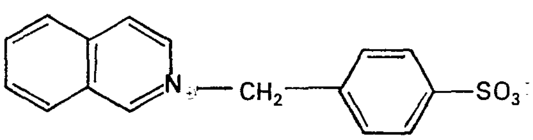
(6);

$(CH_3)_3N^{\oplus}$—$CH_2$—$CO_2^{\ominus}$ (7);

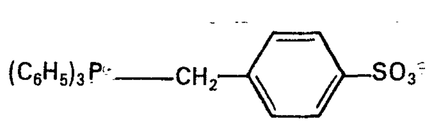
(8).

Erfindungsgemäss werden 0,1 bis 3 Gew.-%, vorzugsweise 0,2 bis 1,5 Gew.-%, bezogen auf die eingesetzten aromatischen Dicarbonsäuren, an Betainen eingesetzt.

Aromatische Dicarbonsäuren entsprechen den folgenden Formeln:

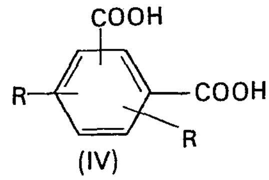
(IV)

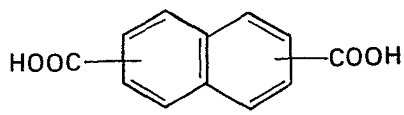
(V)

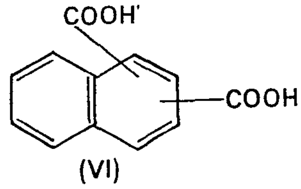
(VI)

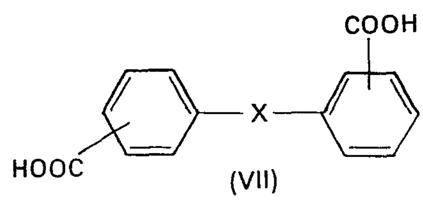
(VII)

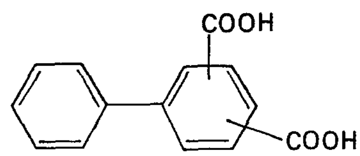
(VIII)

mit R = H, $C_1$–$C_4$-Alkyl oder Halogen (bevorzugt Chlor und Brom)
X = einfache Bindung, -O-, -S-,

$-CH_2-$, 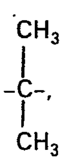 $C_5$–$C_7$ Cycloalkylen.

Auch Gemische können verwendet werden.

Beispielsweise seien genannt:
Phthalsäure, Isophthalsäure, Terephthalsäure, Gemische aus Iso- und Terephthalsäure, Diphensäure und 1,4-Naphthalindicarbonsäure.

Als Lösungs- oder Verdünnungsmittel werden vorzugsweise das oder die (bei Gemischen) aromatischen Dicarbonsäuredichloride eingesetzt, die während der Reaktion gebildet werden. Inerte Verdünnungsmittel, wie aliphatische oder aromatische Kohlenwasserstoffe, Halogen-substituierte aromatische Kohlenwasserstoffe, Halogen-substituierte aliphatische Kohlenwasserstoffe

oder gesättigte aliphatische Ether, können mitverwendet werden. Die Reaktionstemperatur ist im allgemeinen 70 bis 180 °C, vorzugsweise 100 bis 160 °C.

Das Mol-Verhältnis aromatische Dicarbonsäure zu Phosgen ist bevorzugt 1:2 bis 1:2,5, d.h., ein geringer Überschuss an Phosgen ist ratsam, um Verluste zu ersetzen, die bei der Austreibung von $CO_2$- und HCl-Gas aus dem Reaktionsgemisch während der Phosgenierung entstehen.

Das erfindungsgemässe Verfahren kann diskontinuierlich oder kontinuierlich ausgeführt werden. In einer kontinuierlichen Ausführungsform lässt man in einem Reaktionsrohr eine Lösung aus aromatischer Dicarbonsäure, Dicarbonsäuredichlorid und Katalysator nach unten gegen aufströmendes Phosgengas strömen und nimmt am Boden des Reaktionsrohres aromatisches Dicarbonsäuredichlorid und Katalysator ab.

In einer diskontinuierlichen Ausführungsform werden unter Normaldruck aromatische Dicarbonsäure, aromatisches Dicarbonsäuredichlorid und Katalysator vorgelegt. Dann wird unter Rühren auf 70 bis 160 °C erhitzt, wobei sich die aromatische Dicarbonsäure ganz oder teilweise löst. Bei dieser Temperatur werden pro Mol aromatischer Dicarbonsäure 2 bis 2,5 Mol gasförmiges Phosgen eingeleitet.

Nach Entfernen von überschüssigem Phosgen, HCl- und $CO_2$-Gas durch kurzzeitiges Anlegen von Vakuum erhält man einen Rückstand, der neben der Menge an eingesetztem Betainkatalysator zu $\geq$ 99,9% aus aromatischem Dicarbonsäuredichlorid besteht, und der ohne Nachreinigung zu hochmolekularen farblosen Polykondensaten umgesetzt werden kann.

Beispiel 1

203 g (1 Mol) Isophthalsäuredichlorid, 166 g Isophthalsäure (1 Mol) und 0,8 g

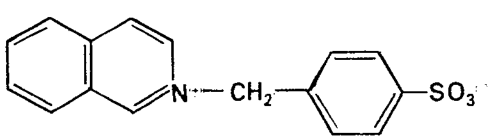

werden im Rundkolben, der mit Thermometer, Rührer und einem durch Kühlsole auf -20 °C gehaltenen Intensivkühler ausgestattet ist, auf 148 °C erhitzt. Bei 148-157 °C wird unter Rühren und Rückflussieden so lange Phosgen eingeleitet, bis im Reaktionsgemisch ein Temperaturabfall auf 146 °C eintritt.

Nach Abkühlen auf 120 °C wird Wasserstrahlkuum angelegt, wodurch überschüssiges Phosgen sowie im Reaktionsgemisch gelöstes HCl- und $CO_2$-Gas entfernt werden.

Ausbeute: 406,5 g eines farblosen Rückstandes aus 0,8 g des eingesetzen Betain-Katalysators und 405,7 g eines 100%igen Isophthalsäuredichlorids (titrimetrisch bestimmt).

Beispiel 2

101,5 g (0,5 Mol) Isophthalsäuredichlorid, 101,5 g (0,5 Mol) Terephthalsäure-dichlorid, 83 g (0,5 Mol)

Isophthalsäure, 83 g (0,5 Mol) Terephthalsäure und 0,8 g

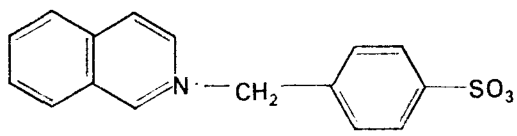

werden, wie im Beispiel 1 beschrieben, mit Phosgen umgesetzt.

Ausbeute: 406,8 g eines farblosen Rückstandes, der zu 0,8 g aus eingesetzten Betain-Katalysator und zu 406 g eines Gemisches aus 100%igem Iso- und Terephthalsäuredichlorid (titrimetrisch bestimmt) besteht.

Beispiel 3

101,5 g (0,5 Mol) Isophthalsäuredichlorid, 101,5 g (0,5 Mol) Terephthalsäuredichlorid, 83 g (0,5 Mol) Isophthalsäure, 83 g (0,5 Mol) Terephthalsäure und 0,8 g

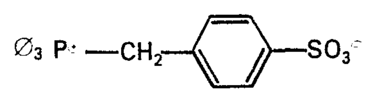

werden, wie im Beispiel 1 beschrieben, mit Phosgen umgesetzt.

Ausbeute: 406 g 99,9%iges Iso- Terephthalsäuredichlorid und 0,8 g eingesetzter Betain-Katalysator.

Beispiel 4

101,5 g (0,5 Mol) Isophthalsäuredichlorid, 101,5 g (0,5 Mol) Terephthalsäuredichlorid, 83 g (0,5 Mil) Isophthalsäure, 83 g (0,5 Mol) Terephthalsäure und 0,3 g

$(CH_3)_3N^{\oplus}-CH_2-CO_2^{\ominus}$

werden, wie im Beispiel 1 beschrieben, mit Phosgen umgesetzt.

Ausbeute: 406 g eines nahezu farblosen Rückstandes aus 0,3 g Katalysator und 405,7 g eines Gemisches aus 100%igem Iso- und Terephthalsäuredichlorid (titrimetrisch bestimmt).

**Patentansprüche**

1. Einstufen-Verfahren zur Herstellung von reinen, aromatischen Dicarbonsäuredichloriden durch Umsetzung eines aromatischen Dicarbonsäuregemisches mit Phosgen in Anwesenheit eines Katalysators, und gegebenenfalls in einem Lösungs- oder Verdünnungsmittel, dadurch gekennzeichnet, dass man als Katalysatoren Betaine verwendet.

2. Verfahren nach Anspruch 1, dadurch gekennzeichnet, dass man als Katalysatoren Sulfobetaine oder Carboxybetaine verwendet.

3. Verwendung der gemäss Anspruch 1 erhaltenen aromatischen Dicarbonsäuredichloride zur Herstellung von Polykondensaten.

## Revendications

1. Procédé à une étape pour la préparation de dichlorures d'acides dicarboxyliques aromatiques purs par réaction d'un acide dicarboxylique aromatique ou d'un mélange d'acides dicarboxyliques aromatiques avec du phosgène en présence d'un catalyseur et éventuellement dans un solvant ou dans un diluant, caractérisé en ce que, comme catalyseurs, on utilise des bétaïnes.

2. Procédé suivant la revendication 1, caractérisé en ce que, comme catalyseurs, on utilise des solfobétaïnes ou des carboxybétaïnes.

3. Utilisation des dichlorures d'acides dicarboxyliques aromatiques obtenus suivant la revendication 1 pour la préparation de polycondensats.

## Claims

1. A single-stage process for the preparation of pure, aromatic dicarboxylic acid dichlorides by reacting an aromatic dicarboxylic acid or an aromatic dicarboxylic acid mixture with phosgene in the presence of a catalyst and optionally in a solvent or diluent, characterised in that betaines are used as catalysts.

2. A process as claimed in Claim 1, characterised in that sulpho-betaines or carboxy-betaines are used as catalysts.

3. The use of the aromatic dicarboxylic acid dichlorides obtained in accordance with Claim 1 in the preparation of polycondensates.